# EUROPEAN PATENT APPLICATION

(11) **EP 4 647 167 A1**
(43) Date of publication of application: **12.11.2025**
(21) Application number: 25172578.4
(22) Date of filing: 25.04.2025
(51) Int. Cl.: B01L 3/00

(54) **A REACTION CONTAINER**

(30) Priority: 08.05.2024 GB 202406381
(71) Applicant: Relco Solutions Limited, Watford WD24 4JP (GB)
(72) Inventor: GILL, Mark, WATFORD, WD24 4JP (GB)
(74) Representative: Serjeants LLP

(57) **Abstract**

A reaction container (10, 32, 34, 36, 40, 48, 50) for a nucleic acid amplification reaction of a biological sample. The reaction container (10, 32, 34, 36, 40, 48, 50) comprises a wall (12) having an inner surface (14) and an outer surface (16). The inner surface (14) defines a cavity (18) for containing the biological sample. At least part of the wall (12) comprises an inductively heatable susceptor material (20) for heating the biological sample.

## Description

### Technical Field

The present disclosure relates generally to reaction containers, and more particularly to reaction containers for nucleic acid amplification reactions.

### Technical Background

Near-patient testing (also known as point-of-care testing) is a diagnostic investigation typically taken at the time of a patient consultation with rapid availability of results from in vitro diagnostics (IVDs), to make immediate and informed decisions about patient care.

IVDs often employ nucleic acid amplification methods, such as Polymerase Chain Reaction (PCR), to detect disease, conditions, and infections.

Using a nucleic acid amplification method, such as PCR, copies of very small amounts of DNA sequences in a biological sample are exponentially amplified in a reaction container in a series of cycles of temperature changes known as thermal cycling. Reagents are subjected to repeated cycles of heating and cooling to permit different temperature-dependent reactions, specifically, DNA melting and enzyme-driven DNA replication.

PCR, for example, employs two main reagents, namely primers (which are short single strand DNA fragments known as oligonucleotides that are a complementary sequence to the target DNA region), and a DNA polymerase.

In a first step of PCR, the two strands of the DNA double helix are physically separated at a high temperature in a process called nucleic acid denaturation. In a second step, the temperature is lowered, and the primers bind to the complementary sequences of DNA. The two DNA strands then become templates for DNA polymerase to enzymatically assemble a new DNA strand from free nucleotides. As PCR progresses, the DNA generated is itself used as a template for replication, setting in motion a chain reaction in which the original DNA template is exponentially amplified.

Thermal cycling of nucleic acid amplification reactions in known reaction containers can be inefficient both in terms of time and energy. Furthermore, it is not always possible control the temperature of biological samples during thermal cycling with precision and accuracy. There is, therefore, a need to mitigate these drawbacks.

### Summary of the Disclosure

According to a first aspect of the present disclosure, there is provided a reaction container for a nucleic acid amplification reaction of a biological sample, the reaction container comprising a wall having an inner surface and an outer surface, the inner surface defining a cavity for containing the biological sample, wherein at least part of the wall comprises an inductively heatable susceptor material for heating the biological sample.

Thermal cycling of nucleic acid amplification reactions in reaction containers according to examples of the disclosure is efficient because the inductively heatable susceptor material has a low thermal mass enabling rapid induction heating in a generated electromagnetic field with low energy requirements.

Furthermore, induction heating of the inductively heatable susceptor material provides precise and accurate temperature control and uniform heating of the biological sample based on the ability to control the power, frequency, and duration of the electromagnetic field with precision and accuracy.

The wall may include a side wall and at least one integral end wall. The at least one integral end wall may have a curved profile. Alternatively, the at least one integral end wall may be substantially planar. The wall may include a side wall and two integral end walls. The two integral end walls may be substantially planar.

The wall may further comprise an electrically insulating material. At least a part of the electrically insulating material may be optically clear.

The reaction container may comprise an electrically insulative layer on at least a part of the inner surface of the wall, wherein the inner surface of the wall is provided by the inductively heatable susceptor material.

Possibly, the at least one integral end wall comprises the inductively heatable susceptor material and the side wall comprises the electrically insulating material. The electrically insulating material may provide the side wall. The at least one integral end wall may consist of the inductively heatable susceptor material. The at least one integral end wall may be formed of the inductively heatable susceptor material. The side wall may consist of the electrically insulating material. The side wall may be formed of the electrically insulating material.

Possibly, the side wall comprises the inductively heatable susceptor material and the at least one integral end wall comprises the electrically insulating material. The inductively heatable susceptor material may provide the side wall. The electrically insulating material may provide the at least one integral end wall. The side wall may consist of the inductively heatable susceptor material. The side wall may be formed of the inductively heatable susceptor material. The at least one integral end wall may consist of the electrically insulating material. The at least one integral end wall may be formed of the electrically insulating material.

The wall may have a side wall, an integral end wall and a closing end wall. The integral end wall may have a curved profile. Alternatively, the integral end wall may be substantially planar. The closing end wall may be substantially planar.

Possibly, the closing end wall comprises the inductively heatable susceptor material, and the side wall and the integral end wall comprise the electrically insulating material. The electrically insulating material may provide the side wall and the integral end wall. The closing end wall may consist of the inductively heatable susceptor material. The closing end wall may be formed of the inductively heatable susceptor material. The side wall and the integral end wall may consist of the electrically insulating material. The side wall and the integral end wall may be formed of the electrically insulating material.

The reaction container may be a tube. The tube may have a common inlet and outlet. The inlet and outlet may be defined by an opening.

Alternatively, the reaction container may be a cartridge. The cartridge may have a separate inlet and outlet. The inlet and outlet may be defined by different openings. The inductively heatable susceptor material may be disposed in the wall substantially between the inlet and the outlet.

In some examples, the inductively heatable susceptor material may be embedded in the electrically insulating material. The inductively heatable susceptor material may be surrounded on all sides by the electrically insulating material.

In other examples, the inductively heatable susceptor material may extend through the whole thickness of the wall thereby providing the inner surface and the outer surface of the at least part of the wall.

According to a second aspect of the present disclosure, there is provided a system comprising:
a reaction container for a nucleic acid amplification reaction of a biological sample, wherein the reaction container comprises a wall having an inner surface and an outer surface, the inner surface defining a cavity for containing the biological sample, wherein at least part of the wall comprises an inductively heatable susceptor material for heating the biological sample; and
an induction coil, wherein the induction coil is configured to generate an alternating electromagnetic field that couples with, and inductively heats, the inductively heatable susceptor material.

The system may comprise a receptacle configured to receive the reaction container and to support the induction coil.

The system may further comprise a cooling arrangement for cooling the biological sample. The cooling arrangement may be a thermoelectric cooler configured to operate by the Peltier effect.

The system may further comprise a non-contact temperature measurement means for measuring the temperature of the biological sample, in use. The non-contact temperature measurement means may comprise a thermocouple. The non-contact temperature measurement means may be configured to detect the temperature of the biological sample.

According to a third aspect of the present disclosure, there is provided a method comprising:
containing a biological sample in a cavity of a reaction container, wherein the reaction container comprises a wall having an inner surface and an outer surface, the inner surface defining the cavity, wherein at least part of the wall comprises an inductively heatable susceptor material; and
generating an alternating electromagnetic field with an induction coil that couples with, and inductively heats, the inductively heatable susceptor material to heat the biological sample until the biological sample reaches a target temperature in accordance with a thermal cycling schedule associated with a nucleic acid amplification reaction.

Possibly, the method comprises receiving, within a receptacle associated with the induction coil, the reaction container.

Possibly, the method comprises cooling the biological sample with a cooling arrangement until the biological sample reaches a target temperature in accordance with the thermal cycling schedule.

According to various, but not necessarily all, embodiments there are provided examples as claimed in the appended claims.

### Brief Description of the Drawings

For a better understanding of various examples that are useful for understanding the detailed description, reference will now be made by way of example only and with reference to the accompanying figures.
Figure 1 is a diagrammatic perspective view of a first example reaction container;
Figure 2 is a diagrammatic cross-sectional view of the first example reaction container;
Figure 3 is a diagrammatic perspective view of a second example reaction container;
Figure 4 is a diagrammatic cross-sectional view of the second example reaction container;
Figure 5 is a diagrammatic perspective view of a third example reaction container;
Figure 6 is a diagrammatic cross-sectional view of the third example reaction container;
Figure 7 is a diagrammatic perspective view of a fourth example reaction container;
Figure 8 is a diagrammatic perspective view of a fifth example reaction container;
Figure 9 is a diagrammatic cross-sectional view of the fifth example reaction container;
Figure 10 is a diagrammatic perspective view of a sixth example reaction container;
Figure 11 is a diagrammatic perspective view of a seventh example reaction container;
Figure 12 is a diagrammatic perspective view of a first example system comprising the fifth example reaction container of Figures 8 and 9 and an induction coil;
Figure 13 is a diagrammatic cross-sectional view of the first example system;
Figure 14 is a diagrammatic perspective view of a second example system comprising the sixth example reaction container of Figure 10 and an induction coil;
Figure 15 is a diagrammatic perspective view of a third example system comprising the seventh example reaction container of Figure 11 and an induction coil;
Figure 16 is a diagrammatic perspective view of a fourth example system comprising the third example reaction container of Figures 5 and 6, an induction coil and a receptacle configured to receive the reaction container and support the induction coil;
Figure 17 is a diagrammatic cross-sectional view of the fourth example system;
Figure 18 is a diagrammatic perspective view of a fifth example system comprising the third example reaction container of Figures 5 and 6, an induction coil, a receptacle configured to receive the reaction container and support the induction coil, and a cooling arrangement; and Figure 19 is a diagrammatic cross-sectional view of the fifth example system.

### Detailed Description of Embodiments

Embodiments of the present disclosure will now be described by way of example only and with reference to the accompanying drawings.

Examples of the disclosure provide a reaction container 10, 32, 34, 36, 40, 48, 50 for a nucleic acid amplification reaction of a biological sample.

As described above, using a nucleic acid amplification method, such as PCR, copies of very small amounts of DNA sequences in a biological sample are exponentially amplified in a series of cycles of temperature changes known as thermal cycling. Reagents are subjected to repeated cycles of heating and cooling to permit different temperature-dependent reactions, specifically, DNA melting and enzyme-driven DNA replication.

PCR, for example, employs two main reagents, namely primers (which are short single strand DNA fragments known as oligonucleotides that are a complementary sequence to the target DNA region), and a DNA polymerase.

In a first step of PCR, the two strands of the DNA double helix are physically separated at a high temperature in a process called nucleic acid denaturation. In a second step, the temperature is lowered, and the primers bind to the complementary sequences of DNA. The two DNA strands then become templates for DNA polymerase to enzymatically assemble a new DNA strand from free nucleotides. As PCR progresses, the DNA generated is itself used as a template for replication, setting in motion a chain reaction in which the original DNA template is exponentially amplified.

A biological sample is any type of biological material derived from a living organism, thereby providing DNA. In use, the reagents (and any buffers) required for a nucleic acid amplification method, along with DNA polymerase, are added to the biological sample.

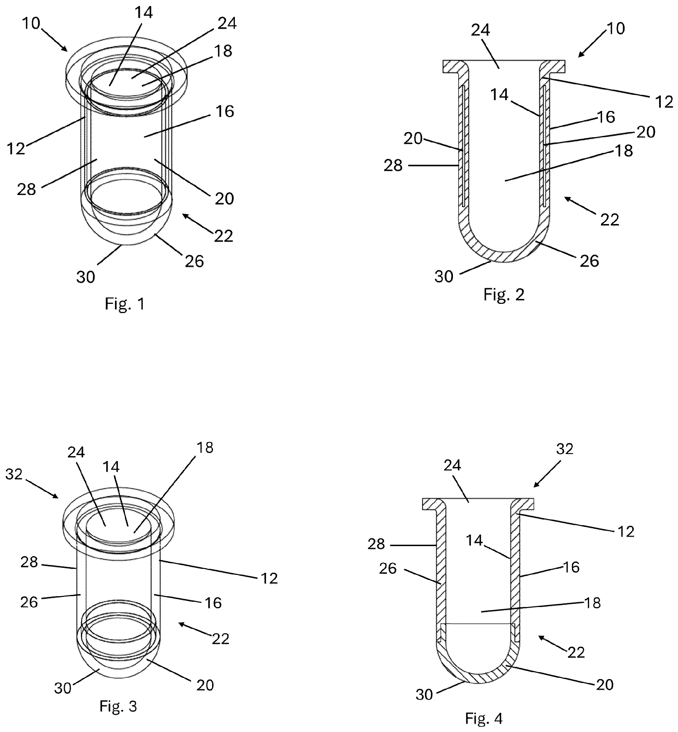

Referring initially to Figures 1 and 2, there is shown a first example reaction container 10.

The first example reaction container 10 comprises a wall 12 having an inner surface 14 and an outer surface 16. The inner surface 14 defines a cavity 18 for containing the biological sample. The cavity 18 is an interior space. The cavity 18 defines an amplification chamber. The wall 12 defines the casing or body of the reaction container 10.

The reaction container 10 is a tube 22 which has an opening 24. The opening 24 provides an inlet into the cavity 18 and outlet from the cavity 18. The reaction container 10 therefore has a common inlet and outlet. The inlet and outlet are defined by the opening 24.

Accordingly, in some examples the wall 12 comprises at least one opening 24 into the cavity 18. In such examples the wall 12 does not enclose the cavity 18.

The wall 12 of the tube 22 includes a side wall 28 and an integral end wall 30. The integral end wall 30 has a curved profile. In the illustrated example, the integral end wall 30 provides an in-use base of the container 10.

In examples of the disclosure, the at least one integral end wall 30 is integrally formed with the side wall 28, for instance, during moulding of the reaction container 10, or otherwise securely bonded to the side wall 28. The at least one integral end wall 30 cannot therefore readily be separated from the side wall 28.

In the illustrated example, the side wall 28 comprises an inductively heatable susceptor material 20 for heating the biological sample (as described in further detail below). Accordingly, at least part of the wall 12 comprises an inductively heatable susceptor material 20.

The inductively heatable susceptor material 20 comprises an electrically conductive material. The inductively heatable susceptor material 20 may comprise one or more, but not limited to, of graphite, molybdenum, silicon carbide, niobium, aluminium, iron, nickel, nickel containing compounds, titanium, mild steel, stainless steel, low carbon steel and alloys thereof, e.g., nickel chromium or nickel copper, and composites of metallic materials.

In use, with the application of an electromagnetic field in its vicinity generated by an induction coil 52 (as described in further detail below), the inductively heatable susceptor material 20 generates heat due to eddy currents and magnetic hysteresis losses resulting in a conversion of energy from electromagnetic to heat. Accordingly, the inductively heatable susceptor material 20 acts, in use, as a heater or heating element. Having generated heat, the inductively heatable susceptor material 20 conducts the heat to the biological sample directly or indirectly (e.g., via an intervening structure). Thus, an external induction circuit including the induction coil 52 facilitates the heating without requiring contact with the biological sample or the reaction container 10.

Examples of the disclosure therefore relate to the use of induction heating in diagnostic testing, such as PCR testing.

The wall 12 of the reaction container 10 further comprises an electrically insulating material 26. The electrically insulating material 26 may be optically clear to allow introduction of a light-source and positioning of an optical detection module at different points around the outside of the reaction container 10. This enables fluorescent (and bioluminescent) detection methods to measure the amount of genetic material such that each time the biological sample is cycled the increase in detected material can be measured. Furthermore, the induction coil 52 can be placed underneath the reaction container 10 such that there is unobstructed access to the side wall 28 for the light source and optical detection module.

The inductively heatable susceptor material 20 is surrounded on all sides by electrically insulating material 26. The inductively heatable susceptor material 20 is therefore embedded in the electrically insulating material 26. The electrically insulating material 26 therefore surrounds the inductively heatable susceptor material 20. The inductively heatable susceptor material 20 may be an in-mould susceptor. In this example, the inductively heatable susceptor material 20 is not therefore in direct contact with the biological sample, which is beneficial if the biological sample (or any of the reagents) are sensitive to the inductively heatable susceptor material 20.

In other examples, the electrically insulating material 26 may surround only a part of the inductively heatable susceptor material 20. In such examples, the inductively heatable susceptor material 20 may be exposed to an extent. An exposed part of the inductively heatable susceptor material 20 may provide a portion of the inner surface 14 and/or outer surface 16 of the at least part of the wall 12. In some examples, the inductively heatable susceptor material 20 comprises a grade of metal, such as 316 Stainless steel, which will not release ions into the biological sample.

In the illustrated example, the inductively heatable susceptor material 20 extends around the circumference of the tube 22. The inductively heatable susceptor material 20 may extend substantially around the circumference of the tube 22. The inductively heatable susceptor material 20 may extend partially around the circumference of the tube 22.

In the illustrated example, the side wall 28 further comprises electrically insulating material 26 in which the inductively heatable susceptor material 20 is embedded. The integral end wall 30 comprises electrically insulating material 26. In this example, the integral end wall 30 does not comprise any inductively heatable susceptor material 20.

In the illustrated example, the wall 12 is substantially formed of the electrically insulating material 26. The inductively heatable susceptor material 20 is therefore a minor component of the wall 12. The wall 12 may consist of only electrically insulating material 26 and inductively heatable susceptor material 20.

Figures 3 and 4 show a second example reaction container 32. The reaction container 32 is similar to the reaction container 10 described above and corresponding components are identified using the same reference numerals. Only the differences relative to the reaction container 10 will hereby be described.

The reaction container 32 is a tube 22. The wall 12 of the tube 22 comprises a side wall 28 and an integral end wall 30. The integral end wall 30 has a curved profile. The integral end wall 30 is therefore curved.

In the illustrated example, the integral end wall 30 comprises the inductively heatable susceptor material 20 and the side wall 28 comprises the electrically insulating material 26. The inductively heatable susceptor material 20 therefore provides the integral end wall 30. The electrically insulating material 26 provides the side wall 28.

In the illustrated example, the integral end wall 30 consists of the inductively heatable susceptor material 20. The integral end wall 30 is therefore formed of inductively heatable susceptor material 20. The side wall 28 consists of the electrically insulating material 26. The side wall 28 is therefore formed of electrically insulating material 26.

The inductively heatable susceptor material 20 extends through the whole thickness of the integral end wall 30. The inductively heatable susceptor material 20 thereby provides the inner surface 14 and the outer surface 16 of the integral end wall 30. In this example, the integral end wall 30 is the at least part of the wall 12 comprising the inductively heatable susceptor material 20.

In the illustrated example, the integral end wall 30 provides an in-use base of the container 32. The inductively heatable susceptor material 20 may be a susceptor base of the tube 22.

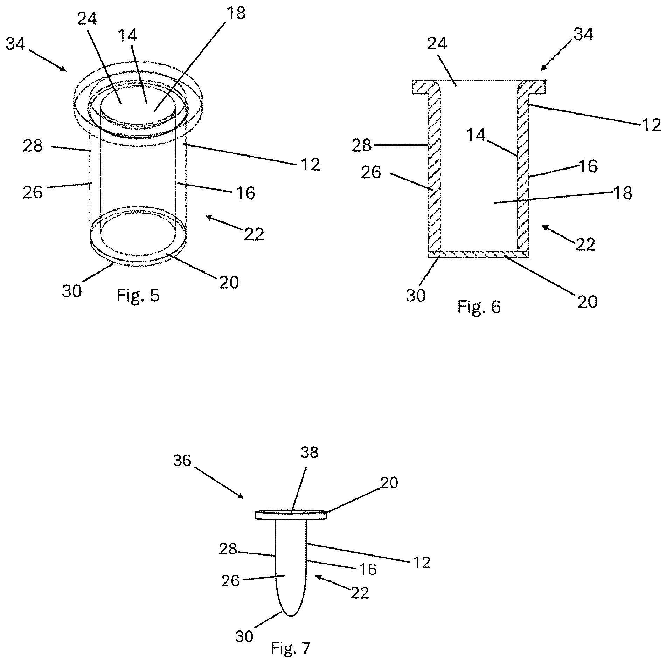

Figures 5 and 6 show a third example reaction container 34. The reaction container 34 is similar to the reaction containers 10, 32 described above and corresponding components are identified using the same reference numerals. Only the differences relative to the reaction containers 10, 32 will hereby be described.

The reaction container 34 is a tube 22. The wall 12 of the tube 22 comprises a side wall 28 and an integral end wall 30. The integral end wall 30 is substantially planar.

In the illustrated example, the integral end wall 30 comprises the inductively heatable susceptor material 20 and the side wall 28 comprises the electrically insulating material 26. The inductively heatable susceptor material 20 therefore provides the integral end wall 30. The electrically insulating material 26 provides the side wall 28.

In the illustrated example, the integral end wall 30 consists of the inductively heatable susceptor material 20. The integral end wall 30 is therefore formed of the inductively heatable susceptor material 20. The side wall 28 consist of the electrically insulating material 26. The side wall 28 is therefore formed of the electrically insulating material 26.

The inductively heatable susceptor material 20 extends through the whole thickness of the integral end wall 30. The inductively heatable susceptor material 20 thereby provides the inner surface 14 and the outer surface 16 of the integral end wall 30. In this example, the integral end wall 30 is the at least part of the wall 12 comprising the inductively heatable susceptor material 20.

In the illustrated example, the integral end wall 30 provides an in-use base of the reaction container 34. The integral end wall 30 may be a susceptor disk.

Figure 7 shows a fourth example reaction container 36. The reaction container 36 is similar to the reaction containers 10, 32, 34 described above and corresponding components are identified using the same reference numerals. Only the differences relative to the reaction containers 10, 32, 34 will hereby be described.

The reaction container 36 is a tube 22. The wall 12 of the tube 22 comprises a side wall 28 and an integral end wall 30. The integral end wall 30 has a curved profile. The integral end wall 30 is therefore curved. The wall 12 further comprises a closing end wall 38. The closing end wall 38 is substantially planar.

The closing end wall 38 closes the opening 24. The wall 12 therefore encloses the cavity 18, i.e., surrounds the cavity 18 on all sides.

In examples of the disclosure, a closing end wall 38 is any part of the wall 12 which is not integrally formed with, or securely bonded to, the side wall 28, for instance, during moulding of the reaction container 36. In some examples, the closing end wall 38 can be separated from the reaction container 36, i.e., the closing end wall 38 is readily separable or removable.

In the illustrated example, the closing end wall 38 comprises the inductively heatable susceptor material 20. The side wall 28 and the integral end wall 30 comprise electrically insulating material 26. The inductively heatable susceptor material 20 therefore provides the closing end wall 38. The electrically insulating material provides the side wall 28 and the integral end wall 30.

In the illustrated example, the closing end wall 38 consists of the inductively heatable susceptor material 20. The closing end wall 38 is therefore formed of the inductively heatable susceptor material 20. The side wall 28 and the integral end wall 30 consist of the electrically insulating material 26. The side wall 28 and the integral end wall 30 are therefore formed of the electrically insulating material 26.

The inductively heatable susceptor material 20 extends through the whole thickness of the closing end wall 38. The inductively heatable susceptor material 20 therefore extends through the whole thickness of the wall 12. The inductively heatable susceptor material 20 thereby provides the inner surface 14 and the outer surface 16 of the closing end wall 38. In this example, the closing end wall 38 is the at least part of the wall 12 comprising the inductively heatable susceptor material 20.

In the illustrated example, the closing end wall 38 provides an in-use base of the reaction container 36. The closing end wall 38 may comprise a foil susceptor, i.e., an induction-sealed foil which closes the tube 22.

In use, a biological sample is loaded into the reaction container 36 through the opening 24. The opening 24 is then closed off, i.e., sealed, by the closing end wall 38. The closing end wall 38 therefore provides a cap or lid. Accordingly, the biological sample, along with the primers etc., are introduced to the reaction container 36 prior to sealing.

In use, the reaction container 36 is inverted so that the closing end wall 38 provides the base of the reaction container 36. In use, the biological sample therefore contacts the closing end wall 38. In such examples, there is no risk of contamination caused by biological sample leaking out of the reaction container 36 through the opening 24.

A variation to the arrangement of Figure 7, is to embed the inductively heatable susceptor material 20, i.e., susceptor, in a press-in cap or screw-on cap.

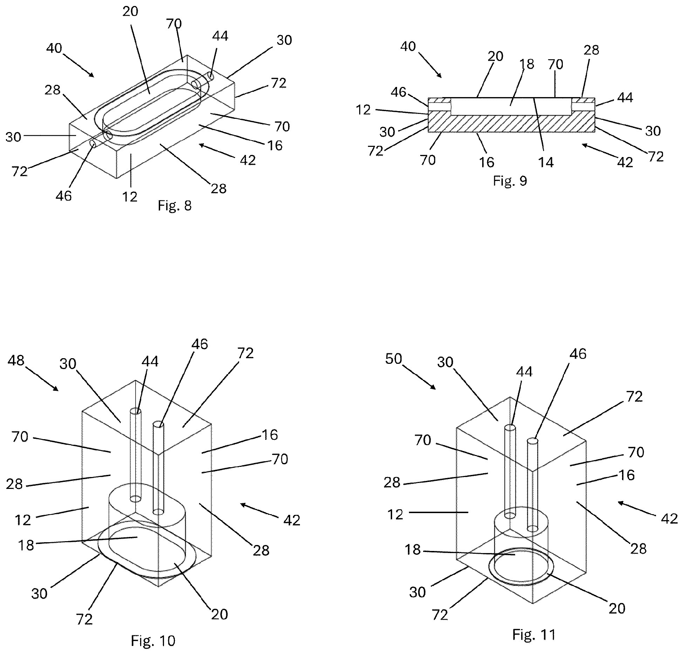

Figures 8 and 9 show a fifth example reaction container 40. The reaction container 40 is similar to the reaction containers 10, 32, 34, 36 described above and corresponding components are identified using the same reference numerals. Only the differences relative to the reaction containers 10, 32, 34, 36 will hereby be described.

The reaction container 40 is a cartridge 42 having a separate inlet 44 and outlet 46. The cartridge 42 has a cuboid shape made up of four larger faces 70 extending around the cartridge 42, and a pair of opposite smaller faces 72.

The wall 12 of the cartridge 42 comprises a side wall 28 and two integral end walls 30. The side wall 28 is provided by the four larger faces 70 of the cuboid. The two integral end walls 30 are provided by the pair of opposite smaller faces 72 of the cuboid.

The integral end walls 30 are substantially planar. The two integral end walls 30 respectively comprise the inlet 44 and outlet 46.

The inductively heatable susceptor material 20 is disposed in the side wall 28 between the inlet 44 and the outlet 46. The inductively heatable susceptor material 20 is therefore disposed in wall 12 between the inlet 44 and the outlet 46.

Accordingly, the side wall 28 comprises the inductively heatable susceptor material 20. The integral end walls 30 comprise the electrically insulating material 26. The inductively heatable susceptor material 20 forms part of the side wall 28. The inductively heatable susceptor material 20 may be a foil susceptor integrated into the side wall 28.

In the illustrated example, the inductively heatable susceptor material 20 is comprised in only one of the four larger faces of the side wall 28. In other examples, the inductively heatable susceptor material 20 may be comprised in more than one of the four larger faces of the side wall 28. In the illustrated example, only a part of one face 70 comprises the inductively heatable susceptor material 20. In other examples, the whole of one or more of the faces 70 may comprise the inductively heatable susceptor material 20. In some examples, the inductively heatable susceptor material 20 may instead be comprised in a part or the whole of one or the other of the integral end walls 30.

The inductively heatable susceptor material 20 extends through the whole thickness of a part of the side wall 28. The inductively heatable susceptor material 20 therefore extends through the whole thickness of the wall 12. The inductively heatable susceptor material 20 thereby provides the inner surface 14 and the outer surface 16 of a part of the side wall 28.

The cartridge 42 design maximizes contact between the biological sample and the inductively heatable susceptor material 20, and having a shallow sample reservoir provides more rapid heating through the biological sample.

Figure 10 shows a sixth example reaction container 48. The reaction container 48 is similar to the reaction container 40 described above and corresponding components are identified using the same reference numerals. Only the differences relative to the reaction container 40 will hereby be described.

The reaction container 48 is a cartridge 42 having a cuboid shape. The cartridge 48 has a separate inlet 44 and outlet 46.

The wall 12 of the cartridge 48 comprises a side wall 28 and two integral end walls 30. The side wall 28 is provided by the four larger faces 70 of the cuboid. The two integral end walls 30 are provided by the pair of opposite smaller faces 72 of the cuboid.

The integral end walls 30 are substantially planar. One of the integral end walls 30 comprises both the inlet 44 and the outlet 46. The other of the integral end walls 30 comprises the inductively heatable susceptor material 20. The inductively heatable susceptor material 20 is therefore comprised in a part of one of the integral end walls 30. Accordingly, the inductively heatable susceptor material 20 forms part of the integral end wall 30.

The inductively heatable susceptor material 20 extends through the whole thickness of a part of the integral end wall 30. The inductively heatable susceptor material 20 therefore extends through the whole thickness of the wall 12. The inductively heatable susceptor material 20 thereby provides the inner surface 14 and the outer surface 16 of a part of the integral end wall 30.

The inductively heatable susceptor material 20 may be a foil susceptor integrated into the integral end wall 30. The inductively heatable susceptor material 20 may take the form of a panel. The inductively heatable susceptor material 20 may have a thickness of about 50 to 100 µm.

The side wall 28 and the other of the integral end walls 30 comprise the electrically insulating material 26.

The inductively heatable susceptor material 20 has a substantially oval shape, in the form of two semicircles joined by a rectangle.

Figure 11 shows a seventh example reaction container 50. The reaction container 50 is similar to the reaction container 48 described above and corresponding components are identified using the same reference numerals. Only the differences relative to the reaction container 48 will hereby be described.

The overall shape and dimensions of the reaction container 50 are different to the reaction container 48. The inductively heatable susceptor material 20 has a substantially circular shape, rather than an oval shape.

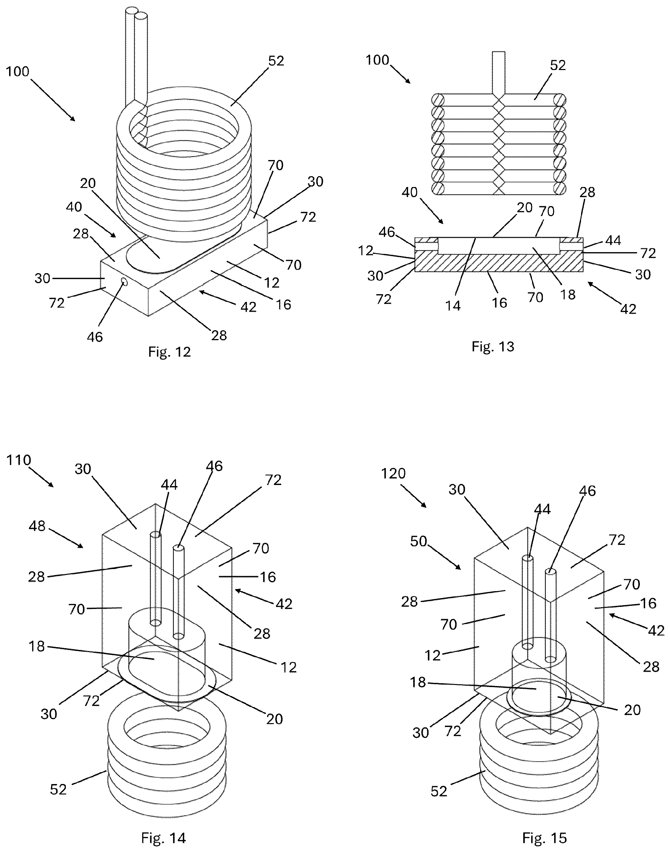

Figures 12 and 13 show a first example system 100 comprising the fifth example reaction container 40 of Figures 8 and 9 and an induction coil 52.

The induction coil 52 is configured to generate an alternating electromagnetic field that couples with, and inductively heats, the inductively heatable susceptor material 20 (as described above). The induction coil 52 is arranged such that the generated alternating electromagnetic field can couple with, and inductively heat, the inductively heatable susceptor material 20. The induction coil 52 is located adjacent to the reaction container 40. The induction coil 52 is operated to induce a current in the inductively heatable susceptor material 20 of the reaction container 40 until the biological sample reaches a target temperature (as described in more detail below).

The induction coil 52 is wound about an axis that is aligned with the reaction container 40. The central axis of the induction coil 52 extends through the reaction container 40.

Figures 14 shows a second example system 110 comprising the sixth example reaction container 48 of Figure 10 and an induction coil 52.

Figures 15 shows a third example system 120 comprising the seventh example reaction container 50 of Figure 11 and an induction coil 52.

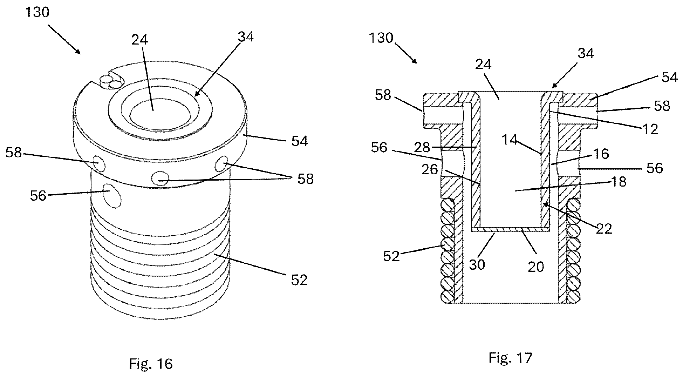

Figures 16 and 17 show a fourth example system 130 comprising the third example reaction container 34 of Figures 5 and 6, an induction coil 52 and a receptacle 54 configured to receive the reaction container 34 and to support the induction coil 52.

In the illustrated example, the receptacle 54 comprises openings 56 for monitoring the emissivity of a biological sample contained in the reaction container 34. In this example, the receptacle 54 further comprises outlets 58 fluidly connectable to a cooling arrangement (not shown).

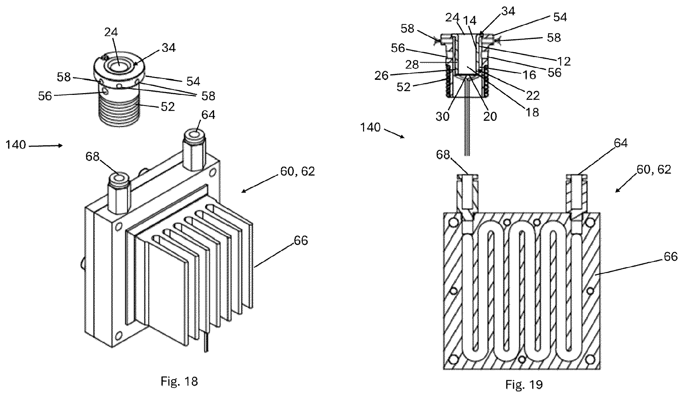

Figures 18 and 19 show a fifth example system 140 comprising the third example reaction container 34 of Figures 5 and 6, an induction coil 52, a receptacle 54 configured to receive the reaction container 34 and to support the induction coil 52, and a cooling arrangement 60 for cooling the biological sample.

In the illustrated example, the cooling arrangement 60 is a thermoelectric cooler 62 configured to operate by the Peltier effect. Although Peltier modules are commonly used for thermocycling, the technology is inefficient in terms of rapid changes from heating to cooling. In examples of the disclosure, Peltier modules are used only for cooling the airflow, thereby avoiding such inefficiencies associated with rapid changes from heating to cooling.

The cooling arrangement 60 comprises an inlet 64, a heat sink 66 and an outlet 68. The outlet 68 is fluidly connected to the receptacle 54.

In use during thermal cycling, to cool the previously heated biological sample, low pressure air is supplied into the cooling arrangement 60 through the inlet 64. The airstream is channelled through the heat sink 66 causing the air temperature to decrease. The cooled airstream exits the cooling arrangement 60 through the outlet 68 and circulates around the reaction container 34 before exiting the receptacle 54 through outlets 58. The cooled airstream reduces the temperature of the biological sample contained in the reaction container 34 in accordance with a thermal cycling schedule. The induction coil 52 can then be operated to again reheat the biological sample in accordance with the thermal cycling schedule.

In other examples, the cooling arrangement 60 could operate with a compressor/heatpump/refrigeration unit as an alternative to a Peltier module.

Systems 100, 110, 120, 130,140 according to examples of the disclosure are thermal cycler systems. Examples of the disclosure provide an increased cycle rate and a reduced cycle time.

In some examples, the system 100, 110, 120, 130, 140 further comprises a non-contact temperature measurement means (not shown) for measuring the temperature of the biological sample, in use. The non-contact temperature measurement means may comprise a thermocouple. The non-contact temperature measurement means may be configured to detect the temperature of the biological sample. Other non-contact means for measuring the temperature of the biological sample may be based on IR or thermal imaging, for example.

The Figures also illustrate a method. The method comprises, containing a biological sample in the cavity 18 of a reaction container 10, 32, 34, 36, 40, 48, 50. The method further comprises generating an alternating electromagnetic field with an induction coil 52 that couples with, and inductively heats, the inductively heatable susceptor material 20 to heat the biological sample until the biological sample reaches a target temperature in accordance with a thermal cycling schedule associated with a nucleic acid amplification reaction, such as PCR.

In some examples, the method comprises receiving, within a receptacle 54 associated with the induction coil 52, the reaction container 10, 32, 34. The method may further comprise cooling the biological sample with a cooling arrangement 60 until the biological sample reaches a target temperature in accordance with the thermal cycling schedule. The cooling arrangement 60 may be associated with the receptacle 54 as described above.

In some examples, the method comprises sequentially heating and cooling the biological sample (as described above) in accordance with the thermal cycling schedule until the nucleic acid amplification reaction has concluded, for instance, because all reagents have been consumed.

In use, changes in resonant frequency can be used to determine the temperature of the inductively heatable susceptor material 20 (i.e., the heating medium) based on algorithms and selection of susceptor material. Accordingly, examples of the disclosure can utilize sensorless temperature control. This can provide high accuracy control and feedback (±0.5°C). In such examples, temperature determination and control can be contactless and/or without the requirement for a temperature sensor. Examples of the disclosure provide improved thermal dynamic performance (i.e., the ability to rapidly heat/cool and hold a temperature at a given set-point without overshoot). Accordingly, the required temperature for optimal amplification is precisely delivered with the capability to adjust the ramp rate and provide variable temperature profiles as desired.

The external induction circuit may comprise an inverter and control algorithms (including a sensorless estimation algorithm) which can deliver complete control over the entire heating process and process reliability.

Based on sensorless temperature control, differential Curie temperature can be used as a means of providing independent confirmation that an expected temperature has been reached. In this case, an inductively heatable susceptor material 20, i.e., a susceptor, could be formed of a laminate of two or more metals, at least one of which has a Curie temperature at a point below 100°C. This is used to perform a calibration function at start-up of the system whereby the susceptor 20 is rapidly heated to the Curie temperature and the resonant frequency shift is used to confirm that this temperature has been reached, this function being important for sensorless temperature control. This would give single-point calibration.

In other examples, a two-point calibration operation may be utilised by using both the Curie point where the susceptor 20 starts to exhibit ferromagnetic properties and the Neel point where it starts to exhibit antiferromagnetic properties. As induction heating is very sensitive to changes in the magnetic properties of the heating element/susceptor 20, the point at which the susceptor 20 changes state can be exactly, i.e., precisely, detected.

The Curie temperature point is when a magnetic material becomes nonmagnetic (ferromagnetic to paramagnetic). The Neel temperature point is when antiferromagnetic materials become paramagnetic.

In practice, the sudden change in susceptibility of the material will lead to a sudden change in some of the state variables of the estimator (in this case it would be a change in observed resistance and/or inductance which could be also indirectly detected by means of a change in current and/or voltage and/or resonant frequency and/or resonant peak amplitude).

A reaction container 10 for such methodology can be constructed out of material(s) with the above-described properties. Without being bound by theory, this could be a layered material where one or multiple layers would have these properties, or it could be an alloy with selected properties.

The Figures also illustrate a method of manufacturing reaction containers 10, 32, 34, 36, 40, 48, 50 and systems 100, 110, 120, 130, 140 according to examples of the disclosure.

There is thus described reaction containers 10, 32, 34, 36, 40, 48, 50, systems 100, 110, 120, 130, 140, and methods with a number of advantages as described above and below.

Thermal cycling of nucleic acid amplification reactions in reaction containers 10, 32, 34, 36, 40, 48, 50 is efficient because the inductively heatable susceptor material 20 has a low thermal mass enabling rapid induction heating in a generated electromagnetic field (and subsequent cooling by de-energising the induction coil 52, and in some examples, by active cooling by a cooling arrangement 60) with low energy requirements.

Furthermore, induction heating of the inductively heatable susceptor material 20 provides precise and accurate temperature control and uniform heating of the biological sample based on the ability to control the power, frequency, and duration of the electromagnetic field with precision and accuracy.

In examples of the disclosure, the heating system can heat a biological sample without requiring conduction of heat through an outer wall 12 of a reaction container 10, 32, 34, 36, 40, 48, 50, thereby reducing the criticality of interface requirements.

In examples of the disclosure, energy transfer is instantaneous giving very rapid temperature change with minimal heat build-up within the system 100, 110, 120, 130, 140 and very low power consumption. In view of the low energy requirements, the system 100, 110, 120, 130, 140 can be battery powered. Accordingly, the system 100, 110, 120, 130, 140 is portable.

The inductively heatable susceptor material 20 requires no physical electrical contact.

Induction heating allows for accurate repetition of a desired thermal cycling schedule. The key parameters (temperature, penetration depth, heat pattern, speed-of-temperature increase) are pre-set in a controller and repeated on each processing cycle.

Although exemplary embodiments have been described in the preceding paragraphs, it should be understood that various modifications may be made to those embodiments without departing from the scope of the appended claims. Thus, the breadth and scope of the claims should not be limited to the above-described exemplary embodiments.

For example, the reaction container 32, 34, 36, 40, 48, 50 may comprise an electrically insulative layer (not shown) on at least a part of the inner surface 14 of the wall 12, where the inner surface 14 of the wall 12 is provided by the inductively heatable susceptor material 20. The inductively heatable susceptor material 20 is not therefore in contact with the biological sample, which is beneficial if any of the reagents are sensitive to the inductively heatable susceptor material 20. The electrically insulative layer therefore acts as a barrier layer between the inductively heatable susceptor material 20 and the biological sample.

For example, systems 130, 140 according to examples of the disclosure may include a plurality of receptacles 54 for receiving reaction containers 10, 32, 34, 36, each respective receptacle 54 including an induction coil 52. In other examples, a plurality of reaction containers 10, 32, 34, 36 may be provided in a strip (for example, six-way or eight-way) or in a plate format (for example, a 96 well plate).

For example, an induction coil shield may be provided around the induction coil 52. The induction coil shield may comprise a layer of ferrite material to prevent the escape of generated electromagnetic field.

For example, reaction containers 10, 32, 34, 36, 40, 48, 50 may include a scannable code encoding a pre-determined thermal cycle needed for a particular diagnostic test or other assay.

For example, temperature sensors such as thermocouples, platinum (PT) sensors, and thermal imaging can be incorporated for process control.

For example, the reagents and primers etc. can be provided in the cavity 18 in a first step, and the biological sample introduced in a second step prior to thermal cycling, or as part of a multichamber cassette/cartridge whereby different solutions are introduced/mixed at different parts of a process along with the thermal cycling (in one or more locations).

For example, the entire nucleic acid amplification reaction process can be undertaken in a self-contained tube 22 or cartridge 42 (i.e., a cassette arrangement), so there is no possibility of contamination.

For example, reaction containers 10, 32, 34, 36, 40, 48, 50 can be provided in combination with a 'lab-on-a-chip' type arrangement.

For example, a metal bead (not shown) can be arranged inside a reaction container 10, 32, 34, 36, 40, 48, 50 and used to agitate the biological sample. In some examples, the induction coil 52 can be utilised to make the metal bead move as desired by modifying the electromagnetic field created by the induction coil 52 (i.e., rapidly switching between higher and lower frequencies).

Any combination of the above-described features in all possible variations thereof is encompassed by the present disclosure unless otherwise indicated herein or otherwise clearly contradicted by context.

Unless the context clearly requires otherwise, throughout the description and the claims, the words "comprise", "comprising", and the like, are to be construed in an inclusive as opposed to an exclusive or exhaustive sense; that is to say, in the sense of "including, but not limited to".

## Claims

1. A reaction container (10, 32, 34, 36, 40, 48, 50) for a nucleic acid amplification reaction of a biological sample, the reaction container (10, 32, 34, 36, 40, 48, 50) comprising a wall (12) having an inner surface (14) and an outer surface (16), the inner surface (14) defining a cavity (18) for containing the biological sample, wherein at least part of the wall (12) comprises an inductively heatable susceptor material (20) for heating the biological sample.

2. A reaction container according to claim 1, wherein the wall (12) has a side wall (28) and at least one integral end wall (30), preferably wherein the at least one integral end wall (30) has a curved profile or is substantially planar.

3. A reaction container according to claim 1 or claim 2, wherein the wall (12) further comprises an electrically insulating material (26), preferably wherein the electrically insulating material (26) is optically clear.

4. A reaction container according to claim 3 when dependent on claim 2, wherein the at least one integral end wall (30) comprises the inductively heatable susceptor material (20) and the side wall (28) comprises the electrically insulating material (26).

5. A reaction container according to claim 1 or claim 3 when dependent only on claim 1, wherein the wall (12) has a side wall (28), an integral end wall (30) and a closing end wall (38), preferably wherein the integral end wall (30) has a curved profile or is substantially planar and the closing end wall (38) is substantially planar.

6. A reaction container according to claim 5 when dependent on claim 3, wherein the closing end wall (38) comprises the inductively heatable susceptor material (20), and the side wall (28) and the integral end wall (30) comprise the electrically insulating material.

7. A reaction container according to claim 6, wherein the closing end wall (38) is formed of the inductively heatable susceptor material (20) and the side wall (28) and the integral end wall (30) are formed of the electrically insulating material (26).

8. A reaction container according to claim 3, wherein the inductively heatable susceptor material (20) is embedded in the electrically insulating material (26), preferably wherein the inductively heatable susceptor material (20) is surrounded on all sides by the electrically insulating material (26).

9. A reaction container according to claim 1, wherein the inductively heatable susceptor material (20) extends through the whole thickness of the wall (12) thereby providing the inner surface (14) and the outer surface (16) of the at least part of the wall (12).

10. A system (100, 110, 120, 130, 140), wherein the system (100, 110, 120, 130, 140) comprises:
a reaction container (10, 32, 34, 36, 40, 48, 50) for a nucleic acid amplification reaction of a biological sample, wherein the reaction container (10, 32, 34, 36, 40, 48, 50) comprises a wall (12) having an inner surface (14) and an outer surface (16), the inner surface (14) defining a cavity (18) for containing the biological sample, wherein at least part of the wall (12) comprises an inductively heatable susceptor material (20) for heating the biological sample; and
an induction coil (52), wherein the induction coil (52) is configured to generate an alternating electromagnetic field that couples with, and inductively heats, the inductively heatable susceptor material (20).

11. A system according to claim 10, wherein the system (130, 140) further comprises a receptacle (54) configured to receive the reaction container (10, 32, 34) and to support the induction coil (52).

12. A system according to claim 10 or claim 11, wherein the system (100, 110, 120, 130, 140) further comprises a cooling arrangement (60) for cooling the biological sample.

13. A method, comprising:
containing a biological sample in a cavity (18) of a reaction container (10, 32, 34, 36, 40, 48, 50), wherein the reaction container (10, 32, 34, 36, 40, 48, 50) comprises a wall (12) having an inner surface (14) and an outer surface (16), the inner surface (14) defining the cavity (18), wherein at least part of the wall (12) comprises an inductively heatable susceptor material (20); and
generating an alternating electromagnetic field with an induction coil (52) that couples with, and inductively heats, the inductively heatable susceptor material (20) to heat the biological sample until the biological sample reaches a target temperature in accordance with a thermal cycling schedule associated with a nucleic acid amplification reaction.

14. A method according to claim 13, wherein the method comprises receiving, within a receptacle (54) associated with the induction coil (52), the reaction container (10, 32, 34).

15. A method according to claim 13 or claim 14, wherein the method comprises cooling the biological sample with a cooling arrangement (60) until the biological sample reaches a target temperature in accordance with the thermal cycling schedule.
